(19) Europäisches Patentamt / European Patent Office / Office européen des brevets

(11) **EP 2 084 511 B1**

(12) **EUROPEAN PATENT SPECIFICATION**

(45) Date of publication and mention
of the grant of the patent:
**09.03.2011 Bulletin 2011/10**

(21) Application number: **07817906.6**

(22) Date of filing: **21.11.2007**

(51) Int Cl.:
*G01N 9/24* (2006.01)          *G01N 23/203* (2006.01)

(86) International application number:
**PCT/DK2007/000510**

(87) International publication number:
**WO 2008/061531 (29.05.2008 Gazette 2008/22)**

(54) **METHOD OF PROVIDING A DENSITY PROFILE OF A PLATE-SHAPED BODY**

VERFAHREN ZUR BEREITSTELLUNG EINES DICHTEPROFILS EINES PLATTENFÖRMIGEN KÖRPERS

PROCÉDÉ PERMETTANT D'OBTENIR LE PROFIL DE DENSITÉ D'UN CORPS DE TYPE PLAQUE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **22.11.2006 DK 200601524**

(43) Date of publication of application:
**05.08.2009 Bulletin 2009/32**

(73) Proprietor: **Force Technology**
**2605 Brøndby (DK)**

(72) Inventors:
• **OLESEN, Finn**
**4623 Lille Skensved (DK)**

• **TELLER, Steen**
**3460 Birkerød (DK)**

(74) Representative: **Siiger, Joergen et al**
**Chas. Hude A/S**
**H.C. Andersens Boulevard 33**
**1780 Copenhagen V (DK)**

(56) References cited:
**DE-A1- 19 622 758      GB-A- 1 602 521**
**US-A- 5 729 582**

• **G. HARDING: "On the sensitivity and application possibilities of a novel Compton scatter imaging system" IEEE TRANSACTIONS ON NUCLEAR SCIENCE, vol. 29, no. 3, 1982, pages 1260-1265, XP001400243 NEW YORK, USA**

**Description**

<u>Technical Field</u>

The invention relates to a method of providing a density profile of a plate-shaped body by means of a radiation source, preferably an X-ray or a γ-ray radiation source, the radiation of which is directed towards the plate-shaped body and scattered therefrom, the radiation scattered by the plate material from a specific depth of the plate-shaped body being taken as a measurement of the density at the respective depth. This relates to plate-shaped bodies, the density of which varies along the thickness of the plate, the density at a particular depth preferably being assumed to be constant, cf. Danish Patent No 171492.

<u>Background</u>

WO 94/27138 discloses the measurement of the density profile of a plate-shaped body by means of a number of γ-ray radiation sources, the radiation being directed from the individual source towards a spot in a predetermined depth of the plate-shaped body and scattered therefrom, the scattered radiation being a measurement of the density at the respective depth. The individual radiation source and the directional detector belonging hereto, however, can only indicate the density at a particular depth. By means of a number of sources/detector pairs it is possible to indicate the density at a corresponding number of different depths, thus obtaining an appropriate number of spots of the density profile. This embodiment is disadvantageous, however, in that it requires a plurality of pairs of sources and detectors and still does not provide any complete density profile.

In order to indicate the entire density profile it is sufficient to have one source and one directional detector, which is movable. Such a movement, however, implies that a measurement (movement from upper side to lower side) takes longer time. Also, the plate must not be moved vertically in a movement as this would influence the measurement of the density profile. This can be difficult to obtain in a practical plate production.

Furthermore, it applies to both above methods that a depth resolution at the magnitude of 0.1 mm, required at measurements on plates with thicknesses at the range of 2-8 mm, cannot be obtained without the measurement time becoming disproportionately long.

GB 1 602 521 describes an arrangement for producing an image of a body section by means of a collimated X-ray radiation source or a collimated γ-radiation source. Scattered radiation is detected through an elongate aperture by means of an array of detector elements. The arrangement ensures that each detector element can only detect scaterred radiation from a specific element section, thereby producing a qualitative representation of the density profile in a section through the body to be imaged. The obtained values may be corrected using a mathematical model and by measuring the total attenuation of the radiation on the way through the body.

<u>Summary of the Invention</u>

The aspect of the invention is to provide a more simple and precise method of determining the density profile of a plate-shaped body.

According to the invention, a method according to claim 1 and an apparatus according to claim 10 are provided.

This method is advantageous in that it is fast and it does not require movable parts. A further advantage is that the entire depth profile is measured at the same time, particularly that the upper side and the lower side of the plate-shaped material are identified simultaneously, whereby the measurement series can be corrected precisely irrespective of the movements of the plate between the partial measurements. Finally, a slot with a narrow width (e.g. 0.1-0.3 mm) is a far more simple construction than complicated collimators for the same depth resolution.

<u>Brief Description of the Drawings</u>

The invention is explained in detail below with reference to the drawings, in which

Fig. 1 shows an apparatus according to the invention for providing a density profile of a plate-shaped body,

Fig. 2 shows the associated electric circuit,

Fig. 3 illustrates the significance of apparatus parameters such as angles and distances in alternative apparatus configurations according to the invention,

Fig. 4 shows an example of the scattering probability as a function of the scattering angle (Klein-Nishina cross section),

Fig. 5 illustrates how the absorption, scattering probability and absorptivity of the scattering material have an influence on the response in the individual detector elements, and

Fig. 6 shows an example of a density profile provided by the method according to the invention compared to the profile obtained by means of laboratory measurements (reference) and a profile of the same plate measured with prior art in scanning.

Detailed description of the invention

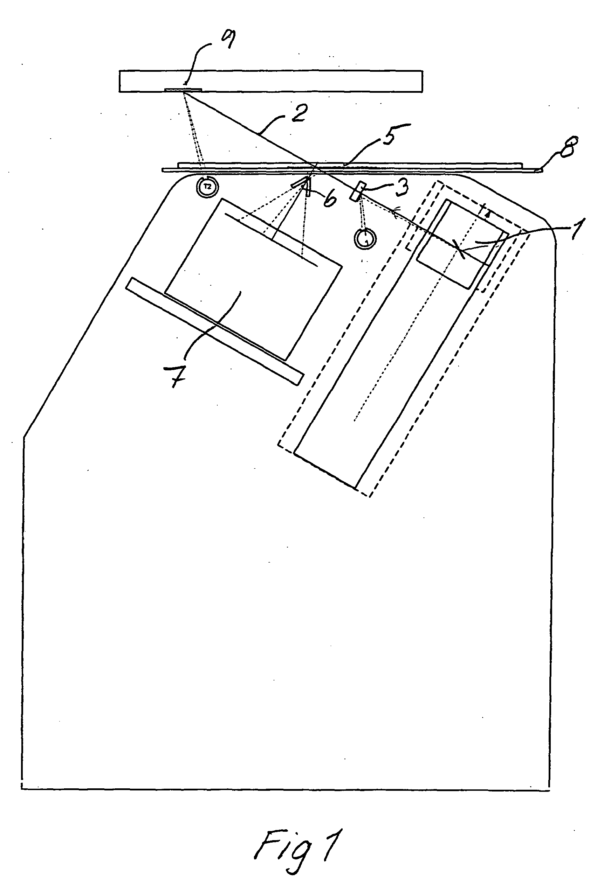

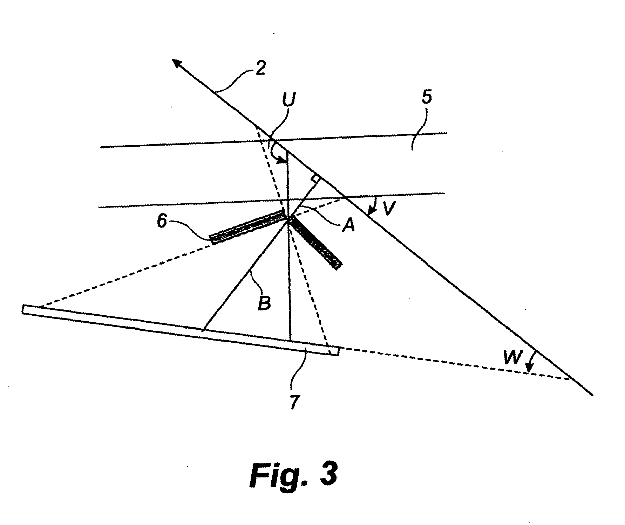

The apparatus according to the invention shown in Fig. 1 for providing a density profile of a plate-shaped body includes an X-ray radiation source 1. The radiation from the source 1 is directed to a collimator 3, from where a collimated radiation is emitted, preferably as a plane fan-shaped bundle of rays 2. The collimated radiation enters the lower side of the stationary or moved plate-shaped body 5 at an inclined angle, the angle of incidence V (c.f. Fig. 3), and is scattered herefrom, the radiation scattered by the plate material from a specific depth of the plate-shaped body 5 being taken as a measurement of the density at the respective depth. According to the invention, the radiation scattered by the plate material at different depths is observed through a narrow, rectangular slot 6 placed substantially perpendicular to the longitudinal direction of the plate 5. This enables observing a scattered radiation from various different depths of the plate material at the same time, utilizing that there is an unambiguous relation between scattering angle and depth. The scattered radiation is registered by means of an array of closely abutting detector elements 7 placed below the slot 6 for determining the density profile. The detector array includes one or more modules, each typically with 50 or 64 individual detectors, e.g. photon counting semiconductor detectors of cadmium telluride or cadmium zinc telluride with widths from less than 0.1 mm to 1 mm. The extension of the individual detector elements in the direction of the rectangular slot, which, with a view to detection of X-ray photons, is desired to be as large as possible, may be 10 mm or more. Possibly, two or more detector arrays may be placed beside each other for hereby increasing the sensitivity and measurement frequency.

Analogous arrays based on registration with silicon diodes of the light, which is caught in a scintillator placed over the diode array 7, are not sensitive enough with today's technology to register the limited number of X-ray photons scattered through the slot, while scintillation detectors based on photomultipliers, such as e.g. a linear gamma camera, typically have too poor a resolution capability (3-5 mm). Besides the detector arrays mentioned here, the technical development may result in more sensitive analogous arrays or scintillation detectors with improved resolution capability or entirely new types of arrays, which may then be utilized in an apparatus according to the invention.

The rectangular slot 6 may consist of two pieces of plate material with high absorptivity such as wolfram or tantalum, possibly lead or gold. The slot 6 results from the two plates forming an angle at e.g. $\pm 45°$ with the perpendicular to the detector array, preferably with the perpendicular to the plane distended from the fan-shaped bundle of rays 2, and shifted towards each other until the desired slot width of typically 0.1-0.3 mm is obtained.

Hereby the slot material opens with a vertical angle at 90° both to the plate 5 irradiated by the fan-shaped bundle of rays 2 and to the detector array 7. If such a large aperture angle is not needed, an improved shielding can be achieved at the sides of the slot by reducing the angle between the two plates from 90° to 60°, at the same time the edges having been ground such as to create an aperture angle at 60° to both sides of the slot 6.

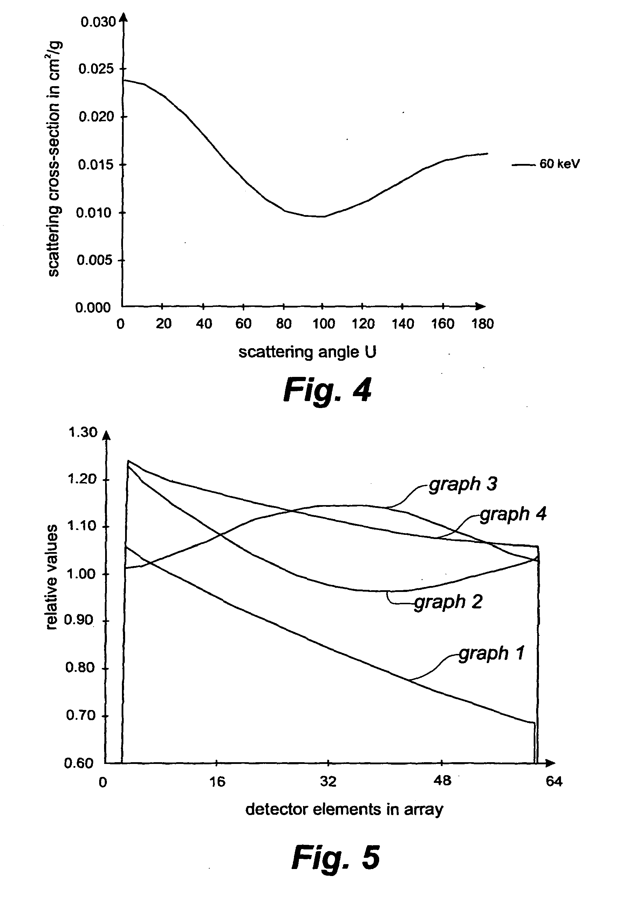

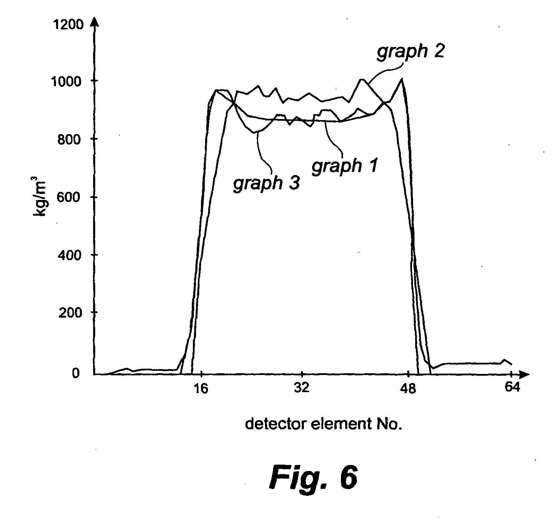

The length of the slot is adapted to the width of the fan-shaped bundle of rays and the length of the detector elements so that all points of scattering of the fan-shaped bundle of rays can reach the full dimensions of the detector element corresponding to the point of scattering through the slot. For instance, the extension of the slot was 25 mm in the model and measurement shown in Figs. 5 and 6, the largest extension of the bundle of rays was approx. 30 mm and the length of the detector elements as mentioned 10 mm.

The mentioned slot does not need to be placed exactly perpendicularly to the scattering direction of the fan-shaped bundle of rays nor lie parallel to the plate. It may also be placed slightly incliningly both in relation to the beam and the plate, as it is possible at least partly to compensate mathematically herefore.

Furthermore, the mentioned slot does not need to be perfectly rectangular. It may also be slightly arched or curved, as it is possible at least partly to compensate mathematically herefore.

Moreover, the mentioned slot does not need to have the same width along its entire extension. Alternatively, the slot may consist of a number of slot parts in continuation of each other. Alternatively, the slot parts may be provided parallel to each other, in this case it is possible mathematically to correct herefore.

The plate 5 may either be placed stationarily above the measuring arrangement or more typically move forward, in the latter case it is appropriate that the plate slides on a bedplate 8 rather than on wheels. The bedplate 8 may either have cuts for radiation passage or be made of a low-absorption material, e.g. a hardwearing plastics type.

In the description relating to Figs. 4 and 5 it is mentioned that for an apparatus of the kind shown in Fig. 1 it is possible mathematically to determine the density profile of the plate-shaped body from the registered scattered radiation in the array of detector elements 7, making use of knowledge of the radiation spectrum, apparatus distance and dimensions, detector parameters as well as the chemical composition of the plate-shaped material 5. In praxis, the described mathematical method will be simplified and be supported by an appropriate number of prior calibration measurements on known plate-shaped bodies for the determination of the density profile from the measured radiation intensities from the individual layers in the plate-shaped body 5.

An automatic calibration for different plate thicknesses with widely different density profiles is obtained through an independent measurement of the area density, e.g. by utilizing the radiation screen 9 for the bundle of rays 2 as a reflector, from which scattered radiation or fluorescent radiation is reflected to a detector T2 placed on the same side of the plate-shaped body 5 as the radiation source 1 and the slot 6 and the detector array 7. The emitted radiation is measured by means of a detector T1, either directly in a partial radiation from the source 1 or as shown in Fig. 1 as radiation reflected from the collimator 3. Then the ratio between the emitted radiation and the radiation transmitted twice through the plate 5 is a measure of the area density.

It is well-known that the radiation absorption in plate-shaped materials of 50-75% is optimal for measuring thickness or area weight. Thus for thin plates or a plate-shaped body of slightly absorbing material such as wood or plastics it is appropriate to provide the radiation screen 9 with a fluorescent reflector, e.g. 1 mm tin. Hereby a substantial part of the X-ray photons in the bundle of rays 2 are converted with energies typically between 50 and 150 keV (kilo electrovolt) into fluorescent radiation with lower energies, which, with tin as reflector, are 25-28 keV. These photons are absorbed in the plate to a higher degree, the measurement of the area density with the detector T2 becoming more sensitive and thereby more precise.

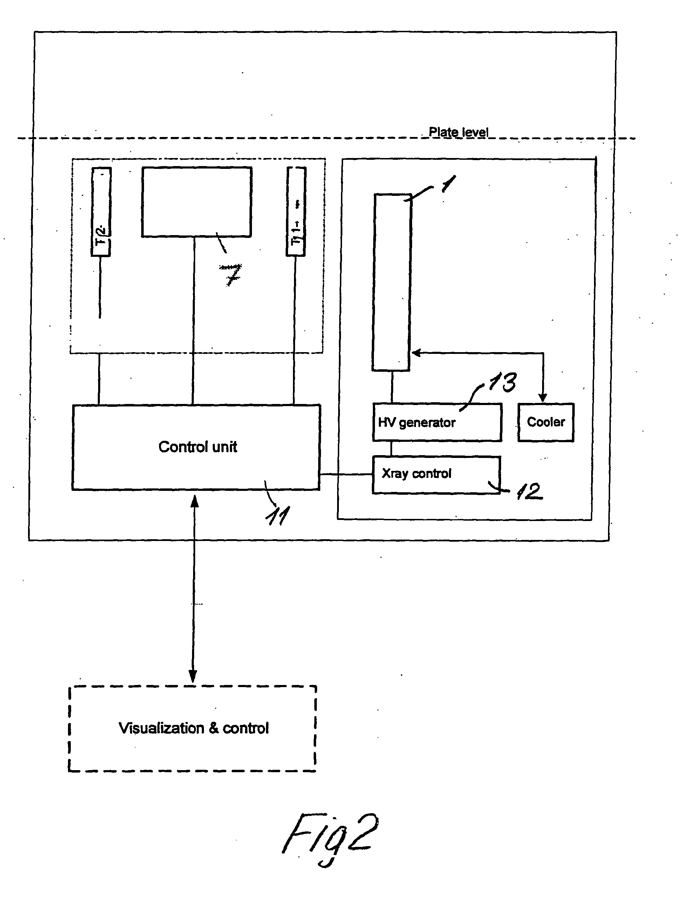

Fig. 2 shows the electric circuit belonging thereto, as it shows the array 7 including sixty-four detector elements, the detectors T1 and T2 and the control unit 11, which is connected to the array 7 and the two detectors T1 and T2. Furthermore, the control unit 11 is connected to an X-ray radiation control 12, which operates a high-voltage generator 13 for the supply of the X-ray source 1.

Fig. 3 illustrates the significance of various geometric parameters such as the angle of incidence V, scattering angle 0, the perpendicular distance A from the fan-shaped bundle of rays 2 to the slot between the slot materials 6 and the distance B from the slot to the detector array 7, measured on the perpendicular to the bundle of rays 2 as well as the angle W between the bundle of rays 2 and the detector array 7. In Fig. 1 the array 7 is parallel to the bundle of rays 2 and the angle W = 0. Thus all parts of the line of the bundle of rays 2 are projected to the array 7 with the same optical or geometrical enlargement B/A irrespective of the scattering angle 0. Considering the triangle and using e.g. the sinus relations, from Fig. 3, in which the angle W # 0, it can be seen the optical amplification varies with Ø as

$$(B/A)*(sin\text{Ø}*cosW)/sin(\text{Ø}-W)$$

Thus, for an apparatus geometry (A, B and W) known and with an unambiguous relation between the detector elements in 7 and the scattering angle Ø, it is possible to carry out the necessary correction of the measurement data.

Fig. 4 shows the differential scattering cross-section for Compton scattering of X-ray or gamma photons as a function of the scattering angle Ø at a photon energy of 60 keV. In the energy area 30-150 keV the absolute values are changed approx. +/-15%, but the form at a minimum around 0 = 90° is not changed. X-ray apparatuses with a high voltage between 60 and 150 kV (kilovolt) emit X-ray photons in an energy spectrum, where the maximum energy in keV is equal to the high voltage in kV, and where the mean value is approx. 50% hereof. Thus, for X-ray photons in this energy area, a model herefore may be a curve substantially equal to the one shown in Fig. 4 and be a valid representation of the scattering probability on the interpretation of measuring data.

For an apparatus of the type shown in Fig.1 with an angle W=0, and where the detector elements 7 of the array, all having the width dp, are placed abutting each other, the following formula can be drawn up for scattered radiation D (p,k) from layer no. k of the thickness dx=dp*(A / B)*sin(V) at the depth k*dx of the plate-shaped body, which after the passage of slot 6 is registered in the detector element no. p:

$$\text{(I)} \quad D(p,k) = Do * exp[-Ao*(dx/sin(V))* \sum_{i=1}^{i=k-1} r(i)] * s(\emptyset(k)) * r(k) * Vol(k) * e(\emptyset(k))$$

$$* \; exp[ -A(k)*(dx/sin(\emptyset(k)-V)* \sum_{i=1}^{i=k-1} r(i)]$$

where Do is the intensity of photons prior to scattering in layer no. k, - the first exponential expression is attenuation to layer no. k, - the second exponential expression is attenuation of scattered radiation with a scattering angle $\emptyset(k)$ from layer no. k in the direction towards the slot 6, - $s(\emptyset(k))$ is the differential scattering cross-section for the scattering angle $\emptyset(k)$, cf. the description for Fig.4, either at a given photon energy or a means for an X-ray spectrum, - r(k) is the density in layer no. k, - Vol(k) is the scattering volume and $e(\emptyset(k))$ is a geometry factor, which takes the distance from the scattering point in layer no. k to the slot into account as well as the apparent size of the slot for different scattering angles.

Do decreases upon increasing k, but at the same time Vol(k) increases correspondingly, Vol(k) equalling the length dx/sin V multiplied with the width and height of the fan-shaped bundle of rays 2. Thus Do*Vol(k) can be replaced by c*lo, where c is a constant, and where lo is the intensity of photons prior to attenuation in the plate-shaped body, for X-ray based radiation sources, possibly corrected for variations in the photon flux from the radiation source 1 by means of T1, cf. Figs. 1 and 2.

The exponential elements include the absorption coefficients Ao and A(k). The coefficients depend on the chemical composition of the plate material and the photon energy. Ao is the absorption coefficient for the ingoing bundle of rays 2, for X-ray radiation averaged over the actual X-ray spectrum. A(k) is the absorption coefficient for the radiation scattered from layer k, and as the photon energy for scattered radiation is unambiguously connected to the energy of non-scattered radiation and the scattering angle $\emptyset(k)$, A(k) can be determined for a given material and a given photon spectrum. Ao and A(k) have the dimension "area per unit mass" and must not be confused with the distance A in Fig. 3, which has the dimension " length".

The distance from the scattering point in layer no. k to the slot 6 is A / cos(90-$\emptyset(k)$), cf. Fig.3, resulting in the intensity of scattered radiation at the slot 6 varying with $(cos(90-\emptyset(k)))^2$, and as the apparent slot area for the scattering angle $\emptyset(k)$ is to be multiplied with $cos(90-\emptyset(k))$ the basic form of $e(\emptyset(k))$ is equal to $(sin\ \emptyset(k))^3$.

The radiation scattered from the plate-shaped body 5 through the slot 6 does not normally cover all detector elements 7 of the array. Thus there are a number of detector elements at each end, where the registered radiation - corrected for background radiation, i.e. registered radiation without the plate-shaped body - is zero except for fluctuations due to the static nature of the radiation. Thus, below a certain low level of registered radiation in the individual detector elements, registered radiation as well as the density in the measuring volume belonging hereto is put equal to zero. Thus in the expression (I) it is correct to put D(p,k) = D(k), the same numbering being applied to the detector elements and measuring volumes.

If the bottom side of the plate-shaped body 5 is assumed to be placed so that the detector element no. q is the first element with registered radiation D(q) above the level mentioned, (I) is found to be:

$$\text{(II)} \quad D(q) = c*lo*s(\emptyset(q)*e(\emptyset(q)*r(q) \quad or \quad r(q) = [D(q) / K(q)]/(c*lo)$$

$$as \; r(k) = 0 \; for \; k < q.$$

Scattering and geometry factors, which solely depend on q, are included in the factor K(q).

For the next element, (I) is found to be:

$$(III) \quad r(q+1) = \{D(q+1)/K(q+1)\}^*\{ exp[F(q+1)^*dx^*r(q)]\}/(c^*Io)$$

where the factor F = Ao/sin(V)+A(q+1)/(sin(Ø(q+1)-V)) for a known chemical composition of the plate-shaped material is an apparatus constant for each detector element, and where r(q) is found in (II).

The following densities in the building-up of the density profile are found by

$$(IV) \quad r(q+i) = \{D(q+i)/K(q+i)\}^*\{exp[F(q+i)^*dx^*\sum_{j=q}^{j=q+i-1} r(j)]\}/(c^*Io)$$

By means of known apparatus constants and material parameters installed in a connected computer or the control unit 11 shown in Fig. 2, a density profile can be deduced for each measured data set in the array 7 without any noticeable time delay. The volume belonging to the first detector element q normally includes both air and plate material, but as F(q+i) for relevant angles V and Ø(q+i) and materials of wood and plastic is of the size 0.5 cm2/g, dx is of the size 0.01 cm and r(q+i) is of the size 1 g/cm3, but possibly with only 10% material in the first volume, the exponential factor may vary between 1.0005 and 1.005 in (III), i.e. a rather insignificant difference. Model calculations show that the unreliability from the density of this first partial layer does not influence the calculation in (IV) to any appreciable extent, but in the worst case only imply a change of the density profile by one detector element.

The mathematical method is normally adjusted and in some cases entirely supplemented with incorporated values from calibration values on laboratory measured plate bodies. Especially material parameters for the plate material and apparatus parameters, such as the geometric reinforcement, are determined by means of calibrated plate bodies.

Scattered radiation from neighbouring layers to the examined layer no. k in the plate-shaped body 5 may contribute to the measured scattered radiation in the detector element corresponding to layer no. k. The nature of these contributions depend on the width of the slot 6, which normally should not be of the same size as the desired resolving power dx / sin(V) in the direction of the fan-shaped bundle of rays 2. The correction required for this ratio is also determined by means of known calibration materials, although it is theoretically possible to correct mathematically through an unfolding procedure.

The previously mentioned independent measurement of area weight, e.g. by means of the auxiliary detectors T1 and T2 shown in Figs. 1 and 2 as well as the radiation screen 9 used as reflector partly determine the choice of correct calibration parameters and partly provide an independent check measurement of the medium density of the plate-shaped material 5, the thickness of the plate being determined by the number of detector elements in the array 7 registering scattered radiation from the plate-shaped material 5.

Fig. 5 shows an example of the elements, which are included in the interpretation of the measuring data of a plate-shaped body, said measuring data being obtained by a method according to the invention: The plate-shaped body is modelled as a 6 mm thick homogeneous wooden plate with a density of 900 kg/m$^3$. The angle of incidence V is 30 °, the detector array with 64 elements, each 0.8 mm wide, is parallel to an X-ray bundle of rays at 115 kV, i.e. the angle W = 0, the optical amplification is 4 (A = 15 and B = 60 mm, cf. Fig. 3) and the slot 0.1 mm. with two tantalum plates á 1 mm and the aperture angle 60°. Graph 1 shows the combined effect of absorption inwards to the scattering point and out of the plate in the direction towards the spot on the detector array corresponding to the scattering point, where the effect of the distance law for radiation during passage of the plate also is taken account of. The thickness of the plate is illustrated in approx. 60 detector elements corresponding to 60*0.8 mm = 48 mm. As the angle of incidence is 30°, the distance of the radiation in the 6 mm thick plate is 6 mm/sin (30) = 12 mm in accordance with the expected optical amplification of 4 times. Graph 2 shows the scattering probability of the angle interval relevant for this plate in this geometry (angle 0 in the interval 68° to 112°), cf. Fig. 4. Graph 3 shows a combined effect of the slot width, the change of the efficient slot width the scattering angle Ø as well as the varies absorption through the obtuse-angled (120°) edges making up the sides of the slot. At scattering angles Ø around 90° radiation from a scattering point with direction towards neighbouring elements to the primary picture element (on the line scattering point-slot) is not absorbed as efficiently as radiation, which does not hit the area around the slot opening as perpendicularly. The overall effect is an increased radiation intensity around the central detector elements. Finally, graph 4 shows the total effect of the 3 graphs. It can be seen, that the raw measuring data is corrected with appropriate choices of geometric parameters, including slot structure, to actual density values for the profile of a plate-shaped material of monotonous and almost linear graphs.

Fig. 6 shows an example of a density profile for a thin plate of grain of wood of 3.1 mm with a medium density of approx. 900 kg/m$^3$ and thin edges with a density of up to approx. 1000 kg/m$^3$ measured partly with laboratory equipment as reference (graph 1), partly with prior art based on measuring of scattered radiation with a movable detector with focussed segment collimator (graph 2) and partly with an apparatus according to the invention (graph 3). The apparatus parameters are the same as the ones mentioned in connection with Fig. 5 with an optical amplification of 4. In conformity herewith, the effective radiation route is seen in the plate, 3.1 mm/sin(30°) = 6.2 mm illustrated in approx. 31 detector elements á 0.8 mm corresponding to 24.8 mm. In this case the raw measuring data have simply been worked up with a linear alignment as well as a setting to the actual area density or basis weight of the plate of 2.8 kg/m$^2$.

Besides the simple method for measuring the area density of the plate-shaped body shown in Fig. 1, whereby the apparatus according to the invention is self-calibrating, area density can be achieved by way of many other methods. For instance, the radiation screen 9 used as reflector in the bundle of rays 2 or parts hereof or in a side beam obtained from the collimator 3 is replaced by a suitable detector for measuring the radiation transmission through the plate. Alternatively, a different measuring system may be applied, either based on radiation absorption or a weight, where data is transmited to the apparatus according to the invention.

The relations between the geometric parameters according to the invention shown in connection with Fig. 3 do not prevent the slot 6 with the detector array 7 from being placed on the opposite side of the plate-shaped material 5, provided that this is considered appropriate. The most substantial parameter, the scattering cross-section as function of the scattering angle 0, is rotation symmetrical about the radiation direction 2 and apart from a correction in the calculation of absorption from the scattering point out of the plate there is no difference. A geometry with an angle V = 90 is also an option, providing the practical possibility of symmetrical placing of two detector arrays with belonging slots both above and below the plate-shaped body.

The plate-shaped body does not need to have constant thickness but may for instance be wedge-shaped or bent as long as it can be presumed that the density profile only varies in one direction within the volume, which covers the incoming and the scattered radiation inclusive of possibly reflected or otherwise transmitted radiation for obtaining area density and inclusive of the extension of the measuring volume due to a possible movement of the plate-shaped body through the measuring section.

In a specific embodiment, an X-ray tube is used, which is operated at a high voltage of 115 V and an amperage of 5 mA. The X-ray tube emits a fan-shaped bundle of rays towards the plate at an angle of 30°. The fan-shaped bundle of rays has a width of approx. 20 mm and a height of 0.2 mm. The photon density in the plate-shaped material can be computed to amount to approx. $2.5 \cdot 10^8$ photons per mm$^2$/sek. The radiation scattered by the plate material is observed through a slot of a width of 0.2 mm. The radiation scattered at different angles is registered by means of an array of 60 detector elements, which are so sensitive that even the individual photon can be registered. The individual detector element has a width of 0.75 mm, and the distance between the detector elements is 0.05 mm. The distance from the plate to the slot is approx. 15 mm, the distance from the plate to the detector elements being approx. 75 mm. The signals registered by the detector elements must be subjected to a software signal processing before the density profile can be determined. As an example, a correction is required so that the scattered radiation does not only depend on the density. It also depends on the scattering angle, cf. Fig. 4. Furthermore, a correction is required, so that a slight attenuation is effected during the transmission of the radiation through the material, cf. Fig. 5.

Taking these conditions into consideration, the density profile shown in graph 3 in Fig. 6 is obtained.

**Claims**

1. Method of providing a density profile of a plate-shaped body (5) by means of a radiation source (1), preferably an X-ray radiation source or a γ-ray radiation source, the radiation of which is directed towards the plate-shaped body (5) and scattered therefrom, the radiation scattered by the plate material from a specific depth of the plate-shaped body (5) being taken as a measurement of the density at the respective depth, wherein the radiation scattered by the plate material at different depths is observed through at least a narrow, substantially rectangular slot (6), which is placed mainly perpendicular to the radiation direction, utilizing that there is an unambiguous relation between scattering angle and depth, said scattered radiation being registered by means of a camera including an array (7) of abuttingly placed detector elements for determining the density profile, **characterised by** further providing an independent measurement of the area density in the plate shaped body (5) by means of a reflector (9) arranged so as to reflect radiation through the plate shaped body (5) to a detector (T2), wherein the reflector (9) comprises a fluorescent reflector, which transforms a part of the X-ray photons in the bundle of rays (2) into fluorescent radiation with lower energy, which is absorbed in the plate to a larger extent, whereby the measurement of the area density becomes more accurate.

2. Method according to claim 1, wherein the reflector (9) is arranged on the opposite side of the plate shaped body (5) than the radiation source (1) and the detector (T2) is placed on the same side of the of the plate shaped body (5)

as the radiation source (1).

3. Method according to claim 1 or 2, wherein an automatic calibration of the density profile for plate thickness and/or different density profiles is provided through the independent measurement of the area density of the plate shaped body.

4. Method according to any of the claims 1 - 3, wherein the fluorescent reflector is a layer of tin converting a part of the X-ray photons in the bundle of rays (2) with energies between 50 and 150 keV to fluorescent radiation with an energy of 25-28 keV.

5. Method according to claim 1, wherein the slot is approx. 0.1-0.3 mm wide.

6. Method according to claim 1, comprising a detector array (7) consisting of at least sixty-four photon counting detector elements.

7. Method according to claim 6, wherein the distance between the detector elements is smaller than 1 mm.

8. Method according to one or more of the preceding claims, wherein the individual detector element is composed of cadmium telluride or cadmium zinc telluride.

9. Method according to one or more of the preceding claims, wherein a mathematical correction is carried out as far as the scattering probability as a function of the scattering angle and the radiant energy is concerned as well as the width of the slot and attenuation in the plate-shaped material are concerned.

10. Apparatus for providing a density profile of a plate-shaped body by means of a radiation source (1), preferably an X-ray or γ-ray radiation source, the radiation of which is directed towards the plate-shaped body (5) and scattered therefrom, the radiation scattered by the plate material from a specific depth of the plate-shaped body (5) being taken as a measurement of the density at the respective depth, wherein a relatively narrow slot (6), is placed substantially perpendicular to the radiation direction for observing the radiation scattered by the plate material to different depths of the plate, and wherein an array (7) of closely abutting detector elements is arranged behind the slot (6) for measuring the radiation scattered at different angles, **characterised in that** the apparatus comprises means for obtaining an independent measurement of the area density, said means comprising a reflector (9) arranged so as to reflect radiation through the plate shaped body (5) to a detector (T2), the reflector comprising a fluorescent reflector, which transforms a part of the X-ray photons in the bundle of rays (2) into fluorescent radiation with lower energy, which is absorbed in the plate to a larger extent, whereby the measurement of the area density becomes more accurate.

11. Apparatus according to claim 10, wherein the reflector (9) is arranged on the opposite side of the plate shaped body (5) than the radiation source (1) and the detector T2 is placed on the same side of the of the plate shaped body (5) as the radiation source (1).

12. Apparatus according to claim 10 or 11, wherein an automatic calibration of the density profile for plate thickness and/or different density profiles is provided through the independent measurement of the area density of the plate shaped body.

13. Apparatus according to any of the claims 10-12, wherein the fluorescent reflector is a layer of tin converting a part of the X-ray photons in the bundle of rays (2) with energies between 50 and 150 keV to fluorescent radiation with an energy of 25-28 keV.

14. Apparatus according to any of the claims 10-13, further comprising a detector (T1) arranged on the same side of the plate shaped body (5) as the radiation source (1), the detector (T1) being configured for measuring the radiation emitted from the radiation source (1).

15. Apparatus according to claim 14, further comprising a control unit (11), which is connected to a camera including the array (7), the detectors (T1, T2), and an X-ray radiation control for operating the radiation source (1).

**Patentansprüche**

1.  Verfahren zum zur Verfügung stellen eines Dichteprofils eines plattenförmigen Körpers (5) mittels einer Strahlungsquelle (1), bevorzugterweise einer Röntgenstrahlungsquelle oder einer γ-Strahlungsquelle, wobei deren Strahlung in Richtung auf den plattenförmigen Körper (5) gerichtet ist und davon gestreut wird, die durch das Plattenmaterial gestreute Strahlung bei einer bestimmten Tiefe des plattenförmigen Körpers (5) als ein Maß der Dichte bei der entsprechenden Tiefe genommen wird, die durch das Plattenmaterial gestreute Strahlung bei unterschiedlichen Tiefen durch zumindest einen engen im Wesentlichen rechteckigen Schlitz (6) beobachtet wird, der hauptsächlich senkrecht zu der Richtung der Strahlung angeordnet ist, wobei ausgenutzt wird, dass eine eindeutige Beziehung zwischen Streuungswinkel und der Tiefe besteht, wobei die gestreute Strahlung mittels einer Kamera registriert wird, die eine Anordnung (7) von aneinander angrenzend angeordneten Detektorelementen zur Bestimmung des Dichteprofils einschließt, **dadurch gekennzeichnet, dass** weiterhin eine unabhängige Messung der Flächendichte in dem plattenförmigen Körper (5) mittels eines Reflektors (9) zur Verfügung gestellt wird, der so angeordnet ist, um Strahlung durch den plattenförmigen Körper (5) zu einem Detektor (T2) zu reflektieren, wobei der Reflektor (9) einen Fluoreszenz-Reflektor umfasst, der einen Teil der Röntgenphotonen in dem Strahlungsbündel (2) in fluoreszente Strahlung mit geringerer Energie umwandelt, die in der Platte in einem größeren Ausmaß absorbiert wird, wodurch die Messung der Flächendichte genauer wird.

2.  Verfahren nach Anspruch 1, wobei der Reflektor (9) auf der der Strahlungsquelle (1) gegenüberliegenden Seite des plattenförmigen Körpers (5) und der Detektor (T2) auf derselben Seite des plattenförmigen Körpers (5) wie die Strahlungsquelle (1) angeordnet ist.

3.  Verfahren nach Anspruch 1 oder 2, wobei eine automatische Kalibrierung des Dichteprofils für die Plattendicke und/ oder verschiedene Dichteprofile durch die unabhängige Messung der Flächendichte des plattenförmigen Körpers zur Verfügung gestellt wird.

4.  Verfahren nach einem der Ansprüche 1-3, wobei der Fluoreszenz-Reflektor eine Lage aus Zinn ist, die einen Teil der Röntgenphotonen in dem Strahlungsbündel (2) mit Energien zwischen 50 und 150 keVin fluoreszente Strahlung mit einer Energie von 25-28 keV umwandelt.

5.  Verfahren nach Anspruch 1, wobei der Schlitz ungefähr 0,1 -0,3 mm breit ist.

6.  Verfahren nach Anspruch 1, umfassend eine Detektoranordnung (7) bestehend aus mindestens vierundsechzig Detektorelementen für das Photonenzählen.

7.  Verfahren nach Anspruch 6, wobei die Distanz zwischen den Detektorelementen kleiner als 1 mm ist.

8.  Verfahren nach einem oder mehreren der voranstehenden Ansprüche, wobei das individuelle Detektorelement aus Cadmiumtellurid oder Cadmiumzinktellurid zusammengesetzt ist.

9.  Verfahren nach einem oder mehreren der voranstehenden Ansprüche, wobei eine mathematische Korrektur durchgeführt wird, insoweit wie die Streuungswahrscheinlichkeit als eine Funktion des Streuungswinkels und der Strahlungsenergie betroffen ist und soweit die Breite des Schlitzes und Abschwächung in dem plattenförmigen Material betroffen sind.

10. Vorrichtung zum zur Verfügung stellen eines Dichteprofils eines plattenförmigen Körpers mittels einer Strahlungsquelle (1), bevorzugterweise einer Röntgenstrahlungsquelle oder einer γ-Strahlungsquelle, wobei deren Strahlung in Richtung auf den plattenförmigen Körper (5) gerichtet ist und davon gestreut wird, die durch das Plattenmaterial gestreute Strahlung bei einer bestimmten Tiefe des plattenförmigen Körpers (5) als ein Maß der Dichte bei der entsprechenden Tiefe genommen wird, wobei ein relativ enger Schlitz (6) im Wesentlichen senkrecht zu der Strahlungsrichtung zum Beobachten der durch das Plattenmaterial gestreuten Strahlung in verschiedenen Tiefen der Platte angeordnet ist, und wobei eine Anordnung (7) von eng angrenzenden Detektorelementen zum Messen der bei verschiedenen Winkeln gestreuten Strahlung hinter dem Schlitz (6) angeordnet ist, **dadurch gekennzeichnet, dass** die Vorrichtung Mittel zum Erhalten einer unabhängigen Messung der Flächendichte umfasst, wobei die Mittel einen Reflektor (9) umfassen, der so angeordnet ist, um Strahlung durch den plattenförmiger Körper (5) zu einem Detektor (T2) zu reflektieren, wobei der Reflektor einen Fluoreszenz-Reflektor umfasst, der einen Teil der Röntgenphotonen in dem Strahlungsbündel (2) in fluoreszente Strahlung mit geringerer Energie umwandelt, die in der Platte in einem größeren Ausmaß absorbiert wird, wodurch die Messung der Flächendichte genauer wird.

**11.** Vorrichtung nach Anspruch 10, wobei der Reflektor (9) auf der der Strahlungsquelle (1) gegenüberliegenden Seite des plattenförmigen Körpers (5) und der Detektor T2 auf derselben Seite des plattenförmigen Körpers (5) wie die Strahlungsquelle (1) angeordnet ist.

**12.** Vorrichtung nach Anspruch 10 oder 11, wobei eine automatische Kalibrierung des Dichteprofils für die Plattendicke und/oder verschiedene Dichteprofile durch die unabhängige Messung der Flächendichte des plattenförmigen Körpers zur Verfügung gestellt wird.

**13.** Vorrichtung nach einem der Ansprüche 10 - 12, wobei der Fluoreszenz-Reflektor eine Lage aus Zinn ist, die einen Teil der Röntgenphotonen in dem Strahlungsbündel (2) mit Energien zwischen 50 und 150 keV in fluoreszente Strahlung mit einer Energie von 25-28 keV umwandelt.

**14.** Vorrichtung nach einem der Ansprüche 10 - 13, weiterhin umfassend einen Detektor (T1), der auf derselben Seite des plattenförmigen Körpers (5) wie die Strahlungsquelle (1) angeordnet ist, wobei der Detektor (T1) zum Messen der durch die von der Strahlungsquelle (1) emittierten Strahlung konfiguriert ist.

**15.** Vorrichtung nach Anspruch 14, weiter umfassend eine Steuerungseinheit (11), die mit einer Kamera verbunden ist, welche die Anordnung (7), die Detektoren(T1, T2)und eine Röntgenstrahlungssteuerung zum Betreiben der Strahlungsquelle (1)einschließt.

**Revendications**

**1.** Procédé pour fournir un profil de densité d'un corps en forme de plaque (5) au moyen d'une source de rayonnement (1), de préférence une source de rayons X ou une source de rayons gamma, dont le rayonnement est dirigé vers le corps en forme de plaque (5) et est dispersé par ce dernier, le rayonnement dispersé par le matériau de la plaque d'une profondeur spécifique du corps en forme de plaque (5) étant pris comme mesure de la densité à la profondeur respective, dans lequel le rayonnement dispersé par le matériau de la plaque à diverses profondeurs est observé à travers au moins une fente étroite sensiblement rectangulaire (6), qui est placée principalement perpendiculaire à la direction du rayonnement, en utilisant le fait qu'il existe une relation sans ambiguïté entre l'angle de dispersion et la profondeur, ledit rayonnement dispersé étant enregistré au moyen d'une caméra comprenant un réseau (7) d'éléments de détecteur disposés en aboutement pour déterminer le profil de densité, **caractérisé par** :

fournir en outre une mesure indépendante de la densité de surface dans le corps en forme de plaque (5) au moyen d'un réflecteur (9) aménagé de manière à réfléchir le rayonnement à travers le corps en forme de plaque (5) vers un détecteur (T2), dans lequel le réflecteur (9) comprend un réflecteur fluorescent, qui transforme une partie des photons de rayons X du faisceau de rayons (2) en rayonnement fluorescent présentant de moindre énergie, qui est absorbé dans la plaque dans une plus grande mesure, de sorte que la mesure de la densité de surface devienne plus précise.

**2.** Procédé selon la revendication 1, dans lequel le réflecteur (9) est aménagé sur le côté du corps en forme de plaque (5) opposé à la source de rayonnement (1) et le détecteur (T2) est placé sur le même côté du corps en forme de plaque (5) que la source de rayonnement (1).

**3.** Procédé selon la revendication 1 ou 2, dans lequel un étalonnage automatique du profil de densité pour l'épaisseur de plaque et/ou de divers profils de densité, est mis en oeuvre par la mesure indépendante de la densité de surface du corps en forme de plaque.

**4.** Procédé selon l'une quelconque des revendications 1 à 3, dans lequel le réflecteur fluorescent est une couche d'étain qui convertit une partie des photons de rayons X du faisceau de rayons (2) présentant des énergies comprises entre 50 et 150 keV en rayonnement fluorescent d'une énergie de 25 à 28 keV.

**5.** Procédé selon la revendication 1, dans lequel la fente présente une largeur d'environ 0,1 à 0,3 mm.

**6.** Procédé selon la revendication 1, comprenant un réseau de détecteurs (7) constitué d'au moins soixante-quatre éléments détecteurs de comptage de photons.

**7.** Procédé selon la revendication 6, dans lequel la distance entre les éléments détecteurs est inférieure à 1 mm.

8. Procédé selon l'une quelconque ou plusieurs des revendications précédentes, dans lequel l'élément détecteur individuel est composé de tellurure de cadmium ou de tellurure de cadmium et de zinc.

9. Procédé selon l'une quelconque ou plusieurs des revendications précédentes, dans lequel une correction mathématique est effectuée pour autant qu'elle concerne la probabilité de dispersion en fonction de l'angle de dispersion et de l'énergie de rayonnement et qu'elle concerne la largeur de la fente et l'atténuation dans le matériau en forme de plaque.

10. Appareil pour fournir un profil de densité d'un corps en forme de plaque au moyen d'une source de rayonnement (1), de préférence une source de rayons X ou de rayons gamma, le rayonnement en question étant dirigé vers le corps en forme de plaque (5) et dispersé par celui-ci, le rayonnement dispersé par le matériau de la plaque d'une profondeur spécifique du corps en forme de plaque (5) étant pris comme mesure de la densité à la profondeur respective, dans lequel une fente relativement étroite (6) est placée sensiblement perpendiculaire à la direction du rayonnement pour observer le rayonnement dispersé par le matériau de la plate à différentes profondeurs de la plate, et dans lequel un réseau (7) d'éléments détecteurs en aboutement étroit est aménagé derrière la fente (6) pour mesurer le rayonnement dispersé sous des angles différents, **caractérisé en ce que** l'appareil comprend des moyens pour obtenir une mesure indépendante de la densité de surface, lesdits moyens comprenant un réflecteur (9) aménagé de manière à réfléchir un rayonnement à travers le corps en forme de plaque (5) vers un détecteur (T2), le réflecteur comprenant un réflecteur fluorescent qui transforme une partie des photons de rayons X du faisceau de rayons (2) en rayonnement fluorescent de moindre énergie, qui est absorbée dans la plaque dans une plus grande mesure, de sorte que la mesure de la densité de surface devienne plus précise.

11. Appareil selon la revendication 10, dans lequel le réflecteur (9) est aménagé sur le côté du corps en forme de plaque (5) opposé à la source de rayonnement (1) et le détecteur (T2) est placé sur le même côté du corps en forme de plaque (5) que la source de rayonnement (1).

12. Appareil selon la revendication 10 ou 11, dans lequel un étalonnage automatique du profil de densité pour l'épaisseur de plaque et/ou de divers profils de densité, est mis en oeuvre par la mesure indépendante de la densité de surface du corps en forme de plaque.

13. Appareil selon l'une quelconque des revendications 10 à 12, dans lequel le réflecteur fluorescent est une couche d'étain qui convertit une partie des photons de rayons X du faisceau de rayons (2) présentant des énergies comprises entre 50 et 150 keV en rayonnement fluorescent d'une énergie de 25 à 28 keV.

14. Appareil selon l'une quelconque des revendications 10 à 13, comprenant en outre un détecteur (T1) aménagé sur le même côté du corps en forme de plaque (5) que la source de rayonnement (1), le détecteur (T1) étant configuré pour mesurer le rayonnement émis par la source de rayonnement (1).

15. Appareil selon la revendication 14, comprenant en outre un unité de commande (11), qui est connectée à une caméra comprenant le réseau (7), les détecteurs (T1, T2) et un dispositif de commande de rayons X pour actionner la source de rayonnement (1).

Fig 1

*Fig2*

**Fig. 3**

**Fig. 4**

**Fig. 5**

**Fig. 6**

**EP 2 084 511 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DK 171492
- WO 9427138 A
- GB 1602521 A